# EUROPEAN PATENT APPLICATION

(11) **EP 4 268 753 A1**
(43) Date of publication of application: **01.11.2023**
(21) Application number: 22176666.0
(22) Date of filing: 01.06.2022
(51) Int. Cl.: A61B 18/18

(54) **TREATMENT DEVICE FOR PERFORMING LIGHT-BASED TREATMENT OPERATIONS**

(30) Priority: 28.04.2022 CN 202210462405; 28.04.2022 CN 202221011186 U
(71) Applicant: Koninklijke Philips N.V., 5656 AG Eindhoven (NL)
(72) Inventor: GU, Wei, Eindhoven (NL); LIN, Xiaoyu, Eindhoven (NL); KONG, Tao, 5656AG Eindhoven (NL); SU, Pu, 5656AG Eindhoven (NL)
(74) Representative: Philips Intellectual Property & Standards

(57) **Abstract**

According to an aspect, there is provided a treatment device (2; 400) for performing light-based treatment operations on or to a subject. The treatment device (2; 400) comprises a U-shaped light source (12; 202; 301; 402) for emitting a light pulse; a light exit window (10; 201) through which the light pulse is emitted from the treatment device (2; 400); and a reflector (302; 403) for reflecting the light pulse towards the light exit window (10; 201). The treatment device (2; 400) is configured to operate with the light source (12; 202; 301; 402) and the reflector (302; 403) in a first configuration (210; 310, 320) for performing hair removal on the subject, and with the light source (12; 202; 301; 402) and the reflector (302; 403) in a second configuration (220; 330) for performing skin rejuvenation on the subject.

## Description

### FIELD OF THE INVENTION

This disclosure relates to a treatment device for performing light-based treatment operations on or to a subject.

### BACKGROUND OF THE INVENTION

Techniques for the removal of unwanted hairs include shaving, electrolysis, plucking, laser and light therapies (known as photoepilation) and injection of therapeutic anti-androgens. Light-based technologies are also used in other types of dermatological treatments, including hair growth reduction and treating acne.

Light-based hair treatments inhibit the growth of hair by exposing the skin to bright flashes or pulses of light, known as intense pulsed light (IPL). Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. The IPL may be generated by a high intensity light source such as a gas discharge lamp (e.g., a Xenon flash lamp). The light penetrates the skin and is absorbed, among other places, in the root of the hair by the pigment melanin. This causes an increase in the temperature of the root of the hair to rise and subsequently the temperature of the surrounding tissue. The generated heat damages the hair follicles, and the growth of the hair is inhibited if the temperature rise is sufficient. This process is known as photothermolysis. When the treatment is repeated in intervals of 2 to 4 weeks, a long-lasting hair reduction is achieved.

Light-based hair removal can be performed using commercially available 'home-use' devices (i.e. consumer devices that are suitable for use by non-specialists) such as the Philips Lumea device. Home-use devices typically use IPL technology at a relatively low fluence (e.g. 4-7 J/cm²) compared to professional devices that use fluences in excess of 10 J/cm². For home-use IPL devices, the treatment area (the area treated by the IPL when the device is held in a single position during use) is typically 3-4 cm², and the pulse rate is typically 1 Hz. With these devices, a whole-body treatment typically takes 10-15 minutes. Feedback from users of these types of devices shows that faster treatment is desired. The treatment speed is dependent on the size of treatment area and the pulse rate. However, for a given lamp, increasing the size of the treatment area results in a lower fluence (light energy per unit area), and thus weaker hair epilation effectiveness.

The total light output of a discharge lamp is proportional to its length. In home-use IPL devices, the size of the discharge lamp is restricted by the size constraints associated with these devices. Thus, in view of the difficulties with increasing the size of the treatment window, and the restriction on the size of the light source, many IPL device manufacturers are working to reduce treatment time by developing flash lamps that operate at higher pulse rates.

IPL can also be used for skin rejuvenation, in which intense light pulses are used to improve the appearance of skin. For example, skin rejuvenation can be used to reduce the visibility of dark spots, redness, wrinkles, scarring, veins and/or broken capillaries. Skin rejuvenation treatment requires higher fluence than hair removal treatment, and so, while there are IPL devices on the market that claim to provide both hair removal and skin rejuvenation, the effectiveness of the skin rejuvenation function is limited by the relatively low fluence provided by existing home-use IPL devices.

### SUMMARY OF THE INVENTION

It is an object of the present disclosure to provide a treatment device that can perform both hair removal and skin rejuvenation effectively. Furthermore, it is desirable for the device to perform faster hair removal treatment than existing devices.

According to the disclosure herein, it is proposed to provide a dual-function treatment device that can operate in two modes: one for performing hair removal treatment and one for performing skin rejuvenation treatment. In particular, it is proposed that a U-shape light source and a reflector of a treatment device has a first configuration for performing hair removal and a second configuration for performing skin rejuvenation.

According to an aspect, there is provided a treatment device for performing light-based treatment operations on or to a subject. The treatment device comprises a U-shaped light source for emitting a light pulse; a light exit window through which the light pulse is emitted from the treatment device; and a reflector for reflecting the light pulse towards the light exit window. The treatment device is configured to operate with the light source and the reflector in a first configuration for performing hair removal on the subject, and with the light source and the reflector in a second configuration for performing skin rejuvenation on the subject.

The use of a U-shape light source can provide a larger treatment area in the first configuration to provide faster hair removal treatment. The fluence level required for hair removal is maintained across this larger treatment area by the use of the U-shaped light source instead of a single straight light source. The compact design of a U-shaped light source can provide significantly higher energy output than a single straight light source without requiring much more space in the IPL device. Thus, the present disclosure provides a more efficient use of space within the device, where space is restricted.

The focusing of the light pulse from the light source into a smaller treatment area in the second configuration provides higher fluence than existing devices, and thus improves the effectiveness of the skin rejuvenation treatment.

Thus, dual functionality is provided with faster hair removal treatment. This is achieved without compromising the compact size of the device or the effectiveness of the skin rejuvenation treatment.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an exemplary treatment device;
Figs. 2(a) and (b) show parts of a treatment device in the first and second configurations respectively according to various embodiments;
Figs. 3(a) and (b) show two exemplary configurations of a treatment device for performing hair removal and Fig. 3(c) shows an exemplary configuration of a treatment device for performing skin rejuvenation;
Figs. 4(a), (b) and (c) illustrate an exemplary mechanism for enabling a treatment device to switch between the first configuration and the second configuration.

### DETAILED DESCRIPTION OF THE EMBODIMENTS

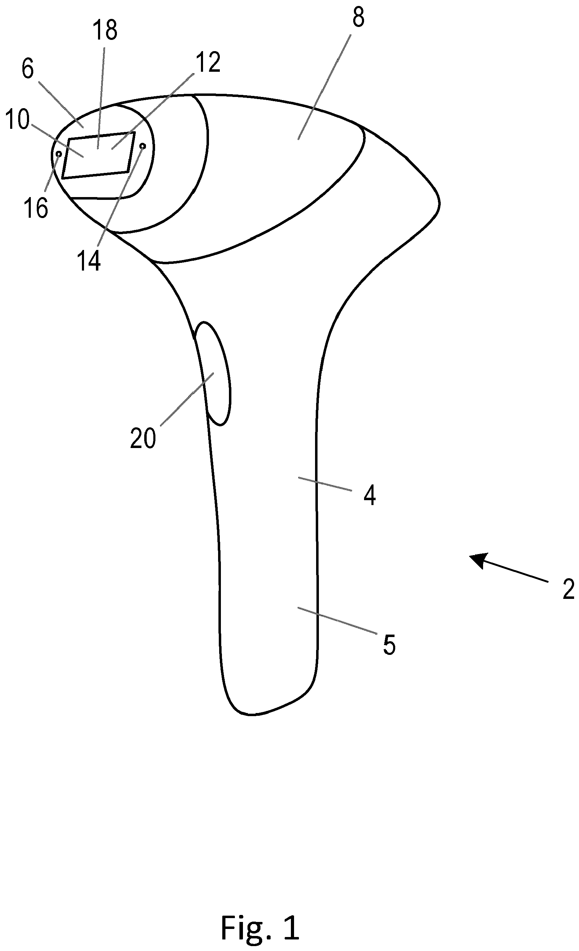

Fig. 1 is an illustration of an exemplary treatment device 2 that can be used to apply a light pulse to an area of skin. It will be appreciated that the treatment device 2 in Fig. 1 is merely presented as an example of a hand-held treatment device 2 that the invention can be used with, and the treatment device 2 is not limited to the form shown in Fig. 1 or to being a hand-held treatment device. The treatment device 2 is for use on a body of a subject (e.g. a person or an animal), and is to be held in one or both hands of a user during use. The treatment device 2 is to perform some treatment operations to hairs and/or skin of the body of the subject using one or more light pulses when the treatment device 2 is close to or in contact with a body part of the subject. The treatment operations include the removal of hairs by laser and/or light therapies (known as a photoepilation treatment or Intense Pulsed Light treatment). The treatment operations also include skin rejuvenation.

As described herein, the treatment device 2 is operated or used by a 'user', and the treatment device 2 is used on a body of a 'subject'. In some cases, the user and the subject is the same person, i.e. the treatment device 2 is held in a hand and used by a user on themselves (e.g. used on the skin on their leg). In other cases, the user and the subject are different people, e.g. the treatment device 2 is held in a hand and used by a user on someone else.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the personal care operation to be performed on the body or skin of the subject at the position that the head portion 6 is in contact with the body or skin.

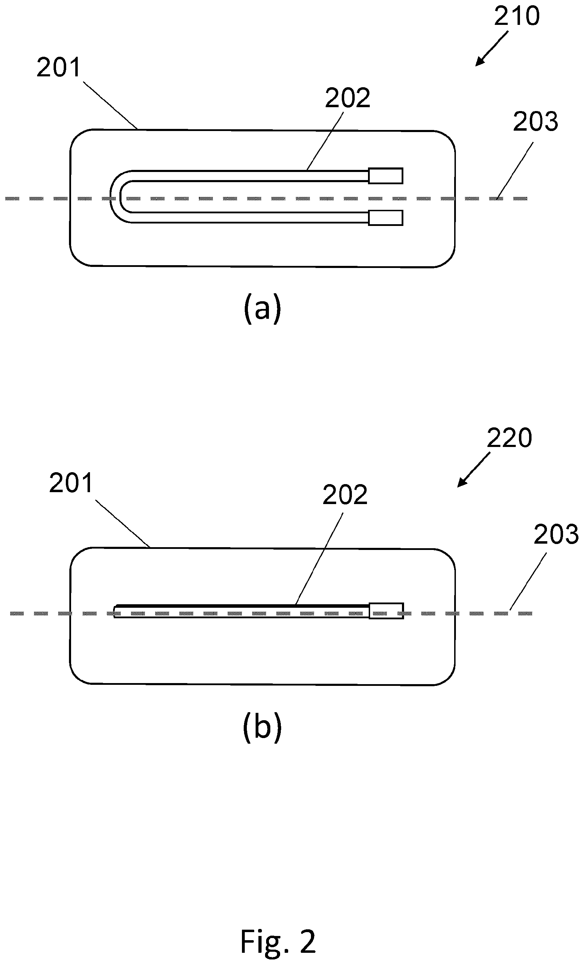

The treatment device 2 is for performing a treatment operation using light pulses. Thus, in Fig. 2 the head portion 6 comprises an aperture 10, also referred to as a light exit window or treatment window, that is arranged in or on the housing 4 so that the aperture 10 can be placed adjacent to or on (e.g. in contact with) the skin of the subject. The treatment device 2 includes a light source 12 that is for generating light pulses that are to be applied to the skin of the subject via the aperture 10 and effect a treatment operation. The light source 12 is arranged in the housing 4 so that the light pulses are provided from the light source 12 through the aperture 10. The aperture/light exit window 10 may be in the form of an opening at the head end 8 of the housing 4, or it may be in the form of a window (including a waveguide) that is transparent or semi-transparent to the light pulses (i.e. the light pulses can pass through the window).

In the exemplary embodiment shown in Fig. 1, the aperture 10 has a generally rectangular shape, which results in a generally rectangular-shaped skin treatment region (also referred to herein as a treatment area) on the skin. It will be appreciated that the aperture 10 can have any other desired shape. For example, the aperture 10 can be square, elliptical, circular, or any other polygonal shape.

The light source 12 can generate light pulses of any suitable or desired wavelength (or range of wavelengths) and/or intensities. For example, the light source 12 can generate visible light, infrared (IR) light and/or ultraviolet (UV) light. The light source 12 can comprise any suitable type of light source, such as a gas discharge lamp, one or more light emitting diodes (LEDs), a laser or lasers, etc.

According to the embodiments herein, the light source is a U-shaped light source, e.g. a U-shaped gas discharge lamp. The gas discharge lamp may comprise a gas in a housing (e.g. a tube), where the gas is typically a noble gas, such as Xenon or Argon, or a mixture of such gases. The gas discharge lamp may be a flash lamp, e.g., a Xenon flash lamp.

The light source 12 can provide light pulses with spectral content in the 560-1200 nanometre (nm) range for a duration of around 2.5 milliseconds (ms), as these wavelengths heat melanin in the hair and hair root by absorption, which puts the hair follicles in a resting phase, preventing hair regrowth.

The light source 12 is configured to provide pulses of light. That is, the light source 12 is configured to generate light at a high intensity for a short duration (e.g. less than 0.1 second). The intensity of the light pulse should be high enough to effect the treatment operation on the skin or body part adjacent the aperture 10.

The illustrated treatment device 2 also includes two skin contact sensors 14, 16 positioned on or in the head portion 6 that are used to determine whether the head portion 6 is in contact with the skin before a light pulse is generated to avoid the light pulse being directed into the eyes of the user or subject.

The illustrated treatment device 2 also includes a skin tone sensor 18 positioned on or in the head portion 6 that is used to determine a skin tone of the skin that the head portion 6 is in contact with.

The illustrated treatment device 2 also includes a user control 20 that can be operated by the user to activate the treatment device 2 so that the head portion 6 performs the required treatment operation on the body of the subject (e.g. the generation of one or more light pulses by the light source 12). The user control 20 may be in the form of a switch, a button, a touch pad, etc. A user control 20 may also be used to switch between different treatment operations.

As noted above, it is desirable for an IPL device to provide both hair removal functionality and skin rejuvenation functionality. It is also desirable to decrease the treatment time for hair removal. However, challenges exist because faster hair removal requires a larger treatment area and/or a faster pulse rate than that used for skin rejuvenation, whereas skin rejuvenation requires higher fluence than that used for hair removal. For a given light source, increasing the treatment area results in lower fluence (i.e. since the emitted light is spread over a larger area). Furthermore, there is limited space within home-use devices that prevents the size of the light source from being significantly increased to enable the required fluence for larger treatment areas. Achieving both a larger treatment area and a higher fluence is therefore challenging.

To address these and other problems, there is provided a treatment device for performing light-based treatment operations on or to a subject. The treatment device could be, for example, the treatment device described with reference to Fig. 1. The treatment device according to some embodiments will now be described with reference to Figs. 2(a), 2(b), 3(a), 3(b), 3(c), 4(a), 4(b) and 4(c).

Figs. 2(a) and (b) show a light exit window 201 and a light source 202 inside a treatment device behind the light exit window 201. The treatment device comprises a U-shaped light source 202 for emitting a light pulse. The U-shaped light source 202 is illustrated in Fig. 2(a). That is, the part of the light source 202 that generates and emits light, is U-shaped. For example, the light source 202 may be a U-shaped flash lamp. In some embodiments, the light source 202 extends generally along a first axis 203. In other words, the first axis 203 is the long axis of the U-shaped light source 202. The first axis 203 lies generally or substantially exactly in a plane that is parallel to the plane of the light exit window 201.

The light pulse is emitted from the treatment device via the light exit window 201. For example, the light exit window 201 could be an opening in the housing of the treatment device, or it could be a transparent or semi-transparent window (including a waveguide). The light exit window 201 could be any shape but is shown in Figs. 2(a) and 2(b) as being generally rectangular. The part of the treatment device comprising the light exit window 201 is placed on or adjacent to the skin during treatment.

The treatment device further comprises a reflector for reflecting the light pulse towards the light exit window 201. The reflector, not shown in Figs. 2(a) and 2(b), is generally positioned around and/or on the opposite side of the light source 202 to the light exit window 201. The reflector acts to reflect the light emitted by the light source 202 towards the light exit window 201.

The treatment device is configured to operate with the light source 202 and the reflector in a first configuration 210 for performing hair removal on the subject, and with the light source 202 and the reflector in a second configuration 220 for performing skin rejuvenation on the subject.

In some embodiments, the light source is rotatable about the first axis 203 to switch between operating in the first configuration and the second configuration. An example of the U-shaped light source 202 in the first configuration 210 for performing hair removal is depicted in Fig. 2(a). An example of the U-shaped light source 202 in the second configuration 220 for performing skin rejuvenation is depicted in Fig. 2(b). In some embodiments, the rotation angle of the U-shaped light source between the first and second configuration is 90 degrees about the first axis 203.

The first axis 203 of the U-shaped light source 202 may be parallel to the light exit window 201 (i.e., parallel to the plane of the light exit window 201) in both the first 210 and second 220 configuration, as shown in Figs. 2(a) and 2(b). In the first configuration 210, the U-shaped light source 202 may be orientated such that a plane comprising the U shape of the U-shaped light source 202 is parallel to the light exit window 201. This is shown in Fig. 2(a). That is, in the first configuration, the U-shaped light source 202 may be orientated such that the two 'arms' of the U are able to emit light directly through the light exit window 201. In the second configuration 220, the U-shaped light source may be orientated such that the plane comprising the U shape of the U-shaped light source is perpendicular to the light exit window 201 (i.e., perpendicular to the plane of the light exit window 201). This is shown in Fig. 2(b). That is, in the second configuration, the U-shaped light source 202 is orientated such that the U-shape is 'side on' to the skin area to be treated.

The reflector may have a different shape in the first configuration 210 to the second configuration 220. In the first configuration 210, the reflector may be configured (e.g., positioned and/or shaped) to reflect light from the light pulse through a first area of the light exit window 201. This first area of the light exit window 201 is equal to (or approximately equal to) the treatment area on the skin of a subject on which the hair removal treatment is performed. In the second configuration 220, the reflector may be configured (e.g. positioned and/or shaped) to reflect light from the light pulse through a second area of the light exit window 201. In some embodiments, the second area is smaller than the first area. The smaller treatment area in the second configuration relative to the first configuration for the same light output by the same light source results in a higher fluence being delivered to the skin in the second configuration compared to the first configuration. The second configuration 220 is thereby able to provide effective skin rejuvenation. On the other hand, the first area can be larger than in conventional treatment devices to provide faster hair removal treatment, since each light pulse is able to treat a larger area, thereby requiring less pulses to cover the body part or body.

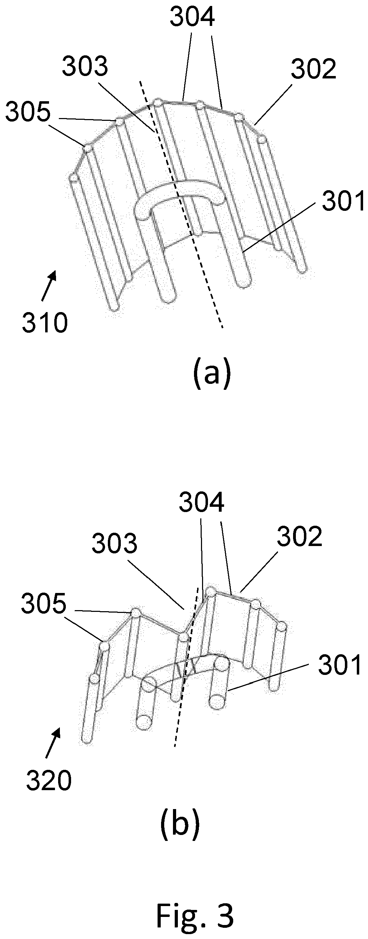

Figs. 3(a) and 3(b) illustrate exemplary configurations of a treatment device for performing hair removal, and 3(c) illustrates an exemplary configuration of a treatment device for performing skin rejuvenation in accordance with some embodiments.

Fig. 3(a) shows the U-shaped light source 301 and the reflector 302 in the first configuration 310 for performing hair removal in accordance with some embodiments. The U-shaped light source 301 extends along the direction of a first axis 303 (shown by the dashed line) and is rotatable about the axis 303 to switch between configurations. The U-shape of the light source increases the light energy emitted per pulse compared to a single straight light source (e.g., a single tube-shaped light source). Advantageously, a U-shaped light source 301 does not take up significantly more space in the head portion of the treatment device than a single straight light source. Indeed, a U-shaped flash lamp can be roughly double the total length of a straight flash lamp whilst fitting into the same sized treatment device. Thus, the U-shaped flash lamp can provide roughly double the total energy output.

The reflector 302 is positioned behind the light source 301 (from the perspective of the light exit window (not shown) and is configured to reflect the light pulse emitted by the light source 301 towards the light exit window. The area of skin that is treated by the direct and reflected light that passes through the light exit window through is referred to herein as the treatment area.

In the first configuration 310, the light source 301 and reflector 302 are configured to deliver the required fluence for hair removal across a larger treatment area than existing devices, and thus provide faster treatment time for a body part that requires multiple light pulses to fully treat. For example, if the light source 301 in the disclosed treatment device according to a specific embodiment is double the length of a light source in an existing treatment device, then the treatment area of the disclosed treatment device can be twice the size whilst still maintaining the required fluence for hair removal treatment. Consequently, the treatment time with the disclosed device according to the specific embodiment can be half that of the existing treatment device.

In some embodiments, the reflector 302 is segmented to allow the shape of the reflector 302 to be changed between configurations. The segments 304 of the reflector 302 extend parallel to the direction of the first axis 303, i.e., along the same direction that the U-shaped light source 301 extends. In the first configuration shown in Fig. 3(a), the segments 304 in the reflector 302 are configured so that they form a generally parabolic shape suitable for directing the light from the U-shaped light source 301 towards the light exit window. In this illustrated embodiment, the segments 304 are connected to neighbouring segments 304 via hinges 305 that enable the segments 304 to move relative to each other, and enable the configuration of the reflector 302 to change.

Fig. 3(b) illustrates a first configuration 320 for performing hair removal according to an alternative embodiment in which a different configuration of the reflector 302 is used. In this alternative first configuration, although the light source 301 has the same shape and orientation as the light source 301 in Fig. 3(a), rather than forming a generally parabolic shape, the segmented reflector 302 forms an 'M' or 'W' shape, with different segments 304 of the U-shaped light source 301 reflecting the light from one of the 'arms' of the U-shaped light source. This shape (combined with the configuration/orientation of the light source 301) improves the uniformity of the reflected light across the treatment area.

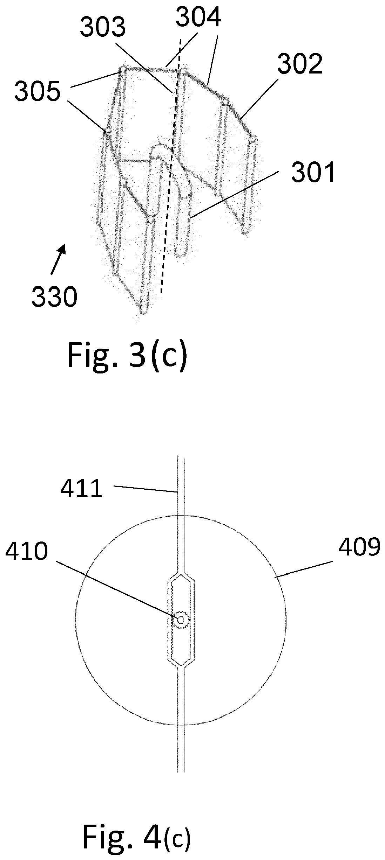

Fig. 3(c) illustrates a second configuration 330 of the treatment device for performing skin rejuvenation according to some embodiments. The light source 301 is rotated 90 degrees about the first axis 303 relative to the orientation of the light source 301 in Figs. 3(a) and 3(b). In this orientation the U-shaped light source can be considered to be 'side on' to the light exit window (whereas in the first configuration the U-shaped light source can be considered to be 'front on' to the light exit window). In some embodiments, the light source 301 is also spaced further from the light exit window compared to its position in the first configuration, e.g., Fig. 2(a) or 2(b). The reflector 302 is configured (e.g., positioned and/or shaped) in the second configuration 330 to reflect the light pulse emitted by the light source 301 onto a smaller treatment area than in the first configuration 310, 320, thereby increasing the fluence in that treatment area compared to the area treated in the first configuration. As in the exemplary first configurations in Figs. 3(a) and 3(b), the segments 304 in the reflector 302 are configured so that they form a generally parabolic shape. However, the parabolic shape is different in view of the different orientation of the U-shaped light source 301, and acts to reflect the light from the U-shape light source 301 towards the light exit window. Concentrating the light from the U-shape light source 301 into a smaller treatment area provides a higher fluence, as required for skin rejuvenation treatment.

The first configurations 310, 320 and second configurations 330 may be such that, for a given light pulse energy, the light pulse in the second configuration 330 delivers a higher fluence at the light exit window than the light pulse in the first configuration 310, 320.

Although in the above embodiments the reflector 302 comprises a plurality of reflecting segments 304 that are connected together via hinges 305 to enable the configuration of the reflector 302 to be changed, those skilled in the art will appreciate that the reflector can have a different construction. For example, the reflector 302 can be a flexible material, e.g. a metallic sheet or a non-reflective flexible material with a reflective coating, that can be manipulated or directed by a suitable mechanism into the required reflector shape for the particular configuration. As another example, the reflector 302 can be formed from a shape-changing material that can be controlled to change between the shapes required for the first and second configurations in response to an electrical signal or other suitable stimulus.

As noted above, the treatment device is configured to operate with the light source and the reflector in a first configuration for performing hair removal on the subject, and in a second configuration for performing skin rejuvenation on the subject. To enable the required treatment mode to be provided, the treatment device comprises a mechanism or other means for enabling the treatment device to switch between configurations. Some exemplary mechanisms are described below, but those skilled in the art will be aware of a variety of different types of mechanisms that can be used to cause the desired configuration change.

In some embodiments, the treatment device comprises a mechanical system for moving the reflector and the light source between the first and second configuration. For example, a system of gear wheels and connection rods may be used to move the reflector and the light source.

In some embodiments, the U-shaped light source may be connected to a main gear wheel that can rotate the light source about the first axis to move the device between the first and second configurations. When the main gear wheel rotates, the light source (e.g. the lamp head) switches between two working modes, i.e. the first and second configuration. In some embodiments, the mechanical system may also translate the light source (e.g. the lamp head) towards the light exit window when it switches to the first configuration for hair removal. This is because, when the light source is rotated from the second configuration into the first configuration, space is created between the light source and the light exit window. By moving the light source closer to the light exit window, the energy of the light pulse is more efficiently transferred to the skin of the subject during treatment. Each connection joint (e.g. hinge) between any two segments of the segmented reflector may be linked to one or more sub-gears via one or more connection rods. These sub-gears can be used to move the reflector between the first and second configurations.

In some embodiments, the treatment device can be changed between the two configurations with one or more manual movements performed by the user of the treatment device. For example, the treatment device can be changed between the two configurations when the user rotates a switch on the treatment device.

In alternative embodiments, the device comprises a motor for moving the reflector and the light source between the first and second configuration. The motor may be controlled by the user, e.g., via a user control button or switch.

In some embodiments, a hair removal filter can be placed in front of the light source in the first configuration, and a skin rejuvenation filter can be placed in front of the light source in the second configuration. The filters may be of different sizes corresponding to the different treatment area sizes and/or the filters can pass different wavelengths of light depending on the treatment mode (hair removal or skin rejuvenation). There may be a mechanical system for ensuring the required filter is in front of the light source in the first and second configuration, but alternatively, the filters can be manually changed (including by removing one filter and replacing it with the filter for the desired configuration).

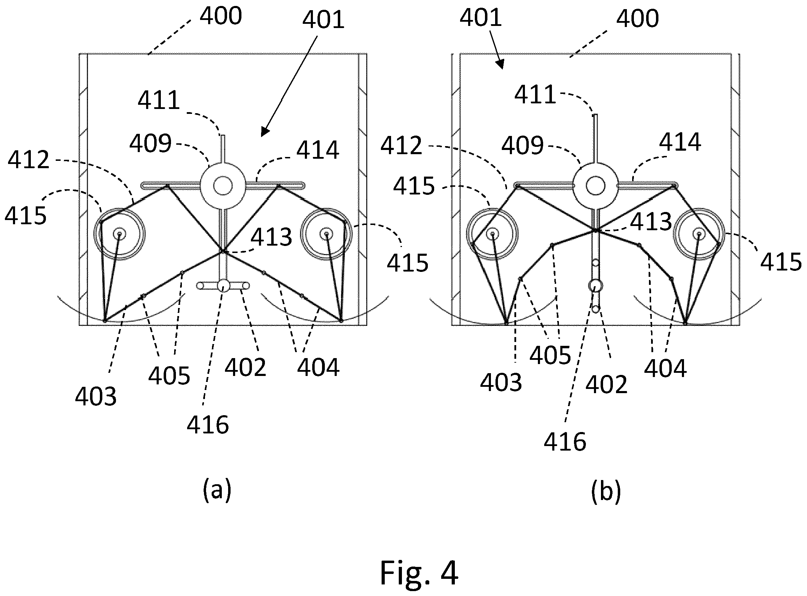

Figs. 4(a), (b) and (c) illustrate an exemplary mechanism for enabling a treatment device to switch between the first configuration and the second configuration. Figs. 4(a) and (b) show a treatment device 400 that includes a mechanism 401 for enabling the treatment device 400 to switch between the first configuration for hair removal (shown in Fig. 4(a)) and the second configuration for skin rejuvenation (shown in Fig. 4(b)). As in the embodiments shown in Fig. 3, the treatment device 400 comprises a rotatable U-shaped light source 402, a reflector 403 that comprises a plurality of segments 404 connected together via hinges 405.

The mechanism 401 comprises a motor 409 that drives the switch between the configurations. As shown in Fig. 4(c), the axle 410 of the motor can rotate one way to drive a centre rod 411 in a first direction (downwards in the perspective of Figs. 4(a) and (b)) to switch to the first configuration, and rotate the opposite way to drive the centre rod 411 in the opposite direction (upwards in the perspective of Figs. 4(a) and (b)). An arrangement of rods 412 is connected to the ends of the reflector 403 and connected to the centre rod 411 at a connection point 413, so that movement of the centre rod 411 by the motor 409 causes the movement of the rods in the rod arrangement 412 and thus a change of the shape of the reflector 403. The movement of rods in the rod arrangement 412 is guided by a guide rail 414, to which two rods are connected, and two wheels 415, to which other rods are connected. The guide rail 414 and wheels 415 guide the movement of the rods 412 to provide the required shape to the reflector 403 as the motor 409 is operated to switch between configurations. As the motor 409 operates to move centre rod 411, it also rotates the light source 90 degrees around centre axis 416.

Therefore, there is disclosed herein a treatment device comprising a U-shaped light source and a reflector. The light source and reflector can be in a first configuration for hair removal, and in a second configuration for skin rejuvenation. The treatment device provides both faster hair removal and effective skin rejuvenation within the same device.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A treatment device (2; 400) for performing light-based treatment operations on or to a subject, the treatment device (2; 400) comprising:
a U-shaped light source (12; 202; 301; 402) for emitting a light pulse;
a light exit window (10; 201) through which the light pulse is emitted from the treatment device (2; 400); and
a reflector (302; 403) for reflecting the light pulse towards the light exit window (10; 201);
wherein the treatment device (2; 400) is configured to operate with the light source (12; 202; 301; 402) and the reflector (302; 403) in a first configuration (210; 310, 320) for performing hair removal on the subject, and with the light source (12; 202; 301; 402) and the reflector (302; 403) in a second configuration (220; 330) for performing skin rejuvenation on the subject.

2. A treatment device (2; 400) as defined in claim 1, wherein the light source (12; 202; 301; 402) extends along a first axis (203; 303) and is rotatable about the first axis (203; 303) to switch between operating in the first configuration (210; 310, 320) and the second configuration (220; 330).

3. A treatment device (2; 400) as defined in claim 2, wherein the light source (12; 202; 301; 402) is rotatable 90 degrees about the first axis (203; 303) to switch between the first configuration (210; 310, 320) and the second configuration (220; 330).

4. A treatment device (2; 400) as defined in claim 2 or 3, wherein the first axis (203; 303) is parallel to the light exit window (10; 201).

5. A treatment device (2; 400) as defined in any of claims 1-4, wherein in the first configuration (210; 310, 320) the U-shaped light source (12; 202; 301; 402) is orientated such that a plane comprising the U shape of the U-shaped light source (12; 202; 301; 402) is parallel to the light exit window (10; 201).

6. A treatment device (2; 400) as defined in any of claims 1-5, wherein in the first configuration (210; 310, 320) the U-shaped light source (12; 202; 301; 402) is orientated such that a plane comprising the U shape of the U-shaped light source (12; 202; 301; 402) is perpendicular to the light exit window (10; 201).

7. A treatment device (2; 400) as defined in any of claims 1-6, wherein the reflector (302; 403) has a different shape in the first configuration (210; 310, 320) to the second configuration (220; 330).

8. A treatment device (2; 400) as defined in any of claims 1-7, wherein the reflector (302; 403) comprises a plurality of segments (304) that are configured to have different positions and/or orientations with respect to each other in the first configuration (210; 310, 320) and in the second configuration (220; 330).

9. A treatment device (2; 400) as defined in any of claims 1-8, wherein in the first configuration (210; 310, 320) the reflector (302; 403) is configured to reflect the light pulse through a first area of the light exit window (10; 201), and in the second configuration (220; 330) the reflector (302; 403) is configured to reflect the light pulse through a second area of the light exit window (10; 201), wherein the second area is smaller than the first area.

10. A treatment device (2; 400) as defined in any of claims 1-9, wherein the U-shaped light source (12; 202; 301; 402) is a U-shaped flash lamp.

11. A treatment device (2; 400) as defined in any of claims 1-10, wherein the light-based treatment operations are Intense Pulsed Light, IPL, treatment operations.

12. A treatment device (2; 400) as defined in any of claims 1-11, wherein the first and second configurations are such that, for a given light pulse energy, the light pulse in the second configuration (220; 330) delivers a higher fluence at the light exit window (10; 201) than the light pulse in the first configuration (210; 310, 320).
